# EUROPÄISCHE PATENTSCHRIFT

(11) **EP 2 139 982 B1**
(45) Veröffentlichungstag und Bekanntmachung des Hinweises auf die Patenterteilung: **16.05.2012**
(21) Anmeldenummer: 08715812.7
(22) Anmeldetag: 16.02.2008
(51) Int. Cl.: C12M 1/12, C12M 1/22, B01D 29/085

(54) **FILTRATIONSEINHEIT UND VERFAHREN ZUR MIKROBIOLOGISCHEN UNTERSUCHUNG FLÜSSIGER PROBEN**
FILTRATION UNIT AND METHOD FOR THE MICROBIOLOGICAL ANALYSIS OF LIQUID SAMPLES
UNITÉ DE FILTRATION ET PROCÉDÉ POUR L'EXAMEN MICROBIOLOGIQUE D'ÉCHANTILLONS LIQUIDES

(30) Priorität: 21.03.2007 DE 102007014082
(43) Veröffentlichungstag der Anmeldung: 06.01.2010
(73) Patentinhaber: Sartorius Stedim Biotech GmbH, 37079 Göttingen (DE)
(72) Erfinder: PFLANZ, Karl, 37130 Gleichen (DE)
(86) Internationale Anmeldenummer: PCT/EP2008/001212
(87) Internationale Veröffentlichungsnummer: WO 2008/113443

(56) Entgegenhaltungen:
- EP-A- 0 239 697
- EP-A- 0 463 897
- EP-A- 0 790 300
- DE-B3-102005 008 220
- FR-A- 2 735 496
- US-A- 4 668 633
- US-A- 5 139 951
- US-A- 5 436 151
- US-A- 6 156 566

## Beschreibung

### Gebiet der Erfindung

Die Erfindung betrifft eine Filtrationseinheit mit einem auf einer Filterunterstützung eines Unterteiles anordenbaren Membranfilter und einem auf das Unterteil aufsetzbaren Aufsatz, sowie eine Nährmedieneinheit zur Aufnahme des Membranfilters der Filtrationseinheit mit einem mit Nährmedium gefüllten Unterteil und einem Deckel.

Weiterhin betrifft die Erfindung ein Verfahren zur mikrobiologischen Untersuchung flüssiger Proben nach vorangegangener Filtration, bei dem ein Membranfilter nach Entfernen eines Aufsatzes zum Aufgießen der flüssigen Probe von einer Filterunterstützung abgehoben und auf einer Oberfläche eines in einem Unterteil einer Nährmedieneinheit angeordneten Nährbodens abgelegt und das Unterteil von einem Deckel abgedeckt wird.

Zur mikrobiologischen Analyse von zu filtrierenden Flüssigkeiten bzw. Proben mit einem Membranfilter wird nach der Filtration der Filter entnommen und beispielsweise in Petrischalen mit einem Nährboden aus Agar eingelegt und mit einem Deckel abgedeckt, wobei die Nährmedieneinheit in einem Brutschrank mehrere Tage bei erhöhter Temperatur lagert. Über den Nährboden erhalten die eventuell vorliegenden Mikroorganismen Nährstoffe, die zum Wachstum anregen, sodass diese bestimmt oder gezählt werden können.

### Stand der Technik

So ist beispielsweise aus der DE 10 2005 008 220 B3 eine Filtrationseinheit mit einem auf einem Unterteil angeordneten Membranfilter und einem auf das Unterteil aufsetzbaren Aufsatz bekannt.

Nachteilig bei dieser Vorrichtung, die sich grundsätzlich bewährt hat, ist, dass ein spezieller aufspreizbarer Entnahmering notwendig ist, mit dem der Membranfilter entnommen und in der Nährmedieneinheit abgelegt wird.

Weiterhin ist aus der DE 198 23 993 B4 eine Einwegvorrichtung zur Keimzahlbestimmung in Flüssigkeiten mit einem Aufgusstrichter, einer mikroporösen Membran mit Membranträger und einer Membranunterstützungseinheit bekannt, wobei der Aufgusstrichter und der Membranträger miteinander fixierbar sind und wobei am Ausgusstrichter mindestens eine Auswerfvorrichtung für den Membranträger vorgesehen ist und der Membranträger und die Auswerfvorrichtung von einer Membranunterstützungseinheit lösbar übergriffen werden.

Nachteilig dabei ist, dass die Filtrationseinheit relativ aufwendig ausgebildet ist und nach der Filtration in einem separaten Schritt über einen definierten Druckpunkt die Filterunterstützung gelöst werden muss. Danach muss in einem zweiten zusätzlichen Schritt die Aufgusstrichter/ Membranträgereinheit über der geöffneten Nährmedieneinheit, d.h. der Agarschale, positioniert und mittels eines zweiten und um 90° versetzten Druckpunktes die Membran auf dem Nährboden abgelegt werden.

Weiterhin ist aus der EP 0 150 775 B1 eine Vorrichtung für die Untersuchung einer Flüssigkeitsprobe mittels Membranfiltration bekannt, bei der der Membranfilter dichtend am Ende einer Hülse befestigt ist. Dieses Hülsenende ist so ausgebildet, dass es als Einsteckaufnahme für einen Medienbehälter dient. Das zweite Hülsenende kann mit einem Deckel dicht verschlossen werden, um die Membran-Medieneinheit verschlossen in den Brutschrank zu geben.

Nachteilig dabei ist eine feste Verbindung zum Aufgusstrichter, die erfordert, dass entweder ein großes Totvolumen akzeptiert wird oder aber zusätzliche Handhabungsschritte zum Verringern des Totvolumens durch Stauchen oder aber auch zum Trennen der Einheit notwendig sind.

### Aufgabenstellung

Aufgabe der vorliegenden Erfindung ist es daher, eine Vorrichtung sowie eine Nährmedieneinheit und ein Verfahren bereitzustellen, bei dem es möglich ist, die Membran ohne den Gebrauch eines zusätzlichen Hilfsmittels nach der Filtration einfach und kostengünstig in die Nährmedieneinheit einzubringen.

Die die Vorrichtung bzw. Filtrationseinheit betreffende Aufgabe wird in Verbindung mit dem Oberbegriff des Anspruches 1 im Wesentlichen dadurch gelöst, dass der Membranfilter einen Verstärkungsrand aufweist und dass der Membranfilter zur Entnahme mit einem Klemmteil eines Deckels einer Nährmedieneinheit verklemmbar und in die Nährmedieneinheit einbringbar ist.

Dadurch, dass der Membranfilter einen Verstärkungsrand aufweist, lässt er sich durch den Deckel einer Nährmedieneinheit, der ein auf den Verstärkungsrand abgestimmtes Klemmteil aufweist, leicht verklemmen, von der Filterunterstützung abheben und in die Nährmedieneinheit einbringen. Dabei können zum einen herkömmliche Filtrationsvorrichtungen verwendet und zum anderen kann ohne zusatzliches Werkzeug der Membranfilter in die Nährmedieneinheit eingebracht werden.

Gemäß einer bevorzugten Ausführungsform der Erfindung ist das Klemmteil als eine ringförmige Innenwandung des Deckels ausgebildet, deren dem Unterteil der Nährmedieneinheit zugewandtes freies Ende klemmend an den Verstärkungsrand anlegbar ist. Vorzugsweise wird dabei das freie Ende der ringförmigen Innenwandung innen an den Verstärkungsrand des Membranfilters angelegt.

Die ringförmige Innenwandung des Deckels ist ihrem Außendurchmesser so auf den Innendurchmesser des Verstärkungsrandes abgestimmt, dass beim Aufsetzen des Deckels auf den Membranfilter die Innenwandung den Verstärkungsrand leicht ausdehnt und so eine klemmende Verbindung zwischen Membranfilter und Deckel hergestellt wird. Der Verstärkungsrand kann auch beispielsweise eine konvexe Ausformung nach innen aufweisen, die in eine korrespondierende konkave Ausformung der Innenwandung des Deckels einrastet. Ein zusätzliches Werkzeug ist nicht notwendig, da der Deckel selbst als Werkzeug dient.

Um für aerobe Keime einen ausreichenden Luftaustausch während der Inkubation zu gewährleisten, ist entweder der Randbereich, über den die klemmende Verbindung erstellt wird, unterbrochen oder der Deckel der Nährmedieneinheit weist eine gegebenenfalls wiederverschließbare zusätzliche Öffnung zur Begasung der Membranoberfläche auf.

Für anaerobe Keime kann eine entsprechende luftdichte, verschlossene Version ohne Öffnung eingesetzt werden.

Die Nährmedieneinheit weist dabei ein schalenförmiges Unterteil auf, das beispielsweise eine Petrischale sein kann, wobei der Deckel auf das Unterteil aufsetzbar ist und in dem Unterteil ein Nährboden, beispielsweise ein Agarboden, angeordnet ist, auf dessen dem Deckel zugewandter Oberseite der im Deckel verklemmte Membranfilter ablegbar ist.

Da der Deckel mit an ihm verklemmten Membranfilter auf die Oberseite des Nährbodens abgelegt wird, ist auch hier ein zusätzliches Werkzeug nicht notwendig.

Erfindungsgemäß ist die Oberseite des Nährbodens konvex gewölbt.

Dadurch, dass der Nährboden bzw. der Agar konvex gewölbt, d.h. in der Mitte höher ist, hat die Membran beim Aufsetzen zuerst mittig Kontakt, sodass beim weiteren Ablegen die Luft nach außen entweichen kann und sich keine Luftblasen bilden. Dies ist wünschenswert, da Luftblasen zwischen Membran und Nährboden sonst eingeschlossen werden könnten, was zu einer Wachstumshemmung führen könnte.

Die Bildung von Luftblasen kann aber auch dadurch vermieden werden, dass der Membranfilter durch einen Überdruck im zwischen ihm und dem Deckel gebildeten Luftraum zu dem Nährboden hin gewölbt ist.

Nach Aufsetzen des Deckels auf das Unterteil liegen der Membranfilter und die Oberseite des Nährbodens eben und abstandsfrei aneinander.

Erfindungsgemäß weist das Unterteil einen Stützring auf, der den Nährboden in seinem unteren Bereich seitlich abstützt.

Nach einer weiteren bevorzugten Ausführungsform der Erfindung ist konzentrisch zur Außenwandung des Unterteiles ein den Nährboden seitlich in einem unteren Bereich abstützender Stützring angeordnet, dessen Innendurchmesser etwa dem Außendurchmesser des Membranfilters entspricht.

Durch den Stützring wird weniger Agar eingesetzt, da nur die Membranfilterfläche mit Agar unterlegt ist. Der Verstärkungsring hat weiter den Vorteil, dass die Oberfläche des Nährbodens bzw. des Agarbodens sich leichter wölben lässt.

Gemäß einer weiteren bevorzugten Ausführungsform der Erfindung ist der Membranfilter aus einer ebenen Membran ausgebildet, auf der zur Verstärkung ihres Randes auf der der Filterunterstützung abgewandten ersten Seite ein den Verstärkungsrand bildender Verstärkungsring angeordnet ist.

Der Verstärkungsring ist dabei vorzugsweise über ein Klebe- oder Siegelverfahren mit der Membran fest verbunden.

Die Aufgabe bezüglich der Nährmedieneinheit wird in Verbindung mit dem Oberbegriff des Anspruches 12 dadurch gelöst, dass der Deckel ein in das Unterteil hineinragendes Klemmteil aufweist, das zur Entnahme des Membranfilters aus der Filtrationseinheit mit einem Verstärkungsrand des Membranfilters verklemmbar ist.

Dadurch, dass der Deckel ein Klemmteil aufweist, lässt sich ein mit einem Verstärkungsrand auf das Klemmteil des Deckels abgestimmter Membranfilter leicht verklemmen, von der Filterunterstützung einer Filtrationseinheit abheben und in das Unterteil der Nährmedieneinheit einbringen. Dabei können zum einen herkömmliche Filtrationsvorrichtungen verwendet und zum anderen kann ohne zusätzliches Werkzeug der Membranfilter in die Nährmedieneinheit eingebracht werden.

Die weitere Aufgabe bezüglich des Verfahrens wird in Verbindung mit dem Oberbegriff des Anspruches 13 dadurch gelöst, dass der Deckel auf den auf der Filterunterstützung liegenden Membranfilter so aufgesetzt wird, dass ein in dem Deckel angeordnetes Klemmteil mit einem Verstärkungsrand des Membranfilters sich verklemmt, und dass der Deckel mit dem Membranfilter von der Filterunterstützung abgehoben und auf das schalenförmige Unterteil der Nährmedieneinheit so aufgesetzt wird, dass die dem Deckel abgewandte Unterseite des Membranfilters auf der dem Deckel zugewandten Oberseite des Nährbodens aufliegt.

Dadurch, dass der Membranfilter mit dem Deckel verklemmt und der Membranfilter mit dem Deckel von der Filterunterstützung abgehoben und auf die Oberseite des in der Nährmedieneinheit angeordneten Nährbodens aufgelegt wird, werden zusätzliche Schritte vermieden und der Membranfilter kann ohne ein zusätzliches Werkzeug in die Nährmedieneinheit verbracht werden.

Gemäß einer bevorzugten Ausführungsform der Erfindung wird der Deckel mit dem geklemmten Membranfilter auf eine Petrischale mit einer Agarplatte als Nährboden aufgesetzt.

Das erfindungsgemäße Verfahren kann mit im Wesentlichen bekannten Vorrichtungen durchgeführt werden, lediglich der Membranfilter und der Deckel der Nährmedieneinheit müssen erfindungsgemäß modifiziert werden. Damit ergibt sich ein einfaches und kostengünstiges Manipulieren des Membranfilters mit der aufgebrachten Probe.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibungen der Zeichnungen

In den Zeichnungen zeigen:

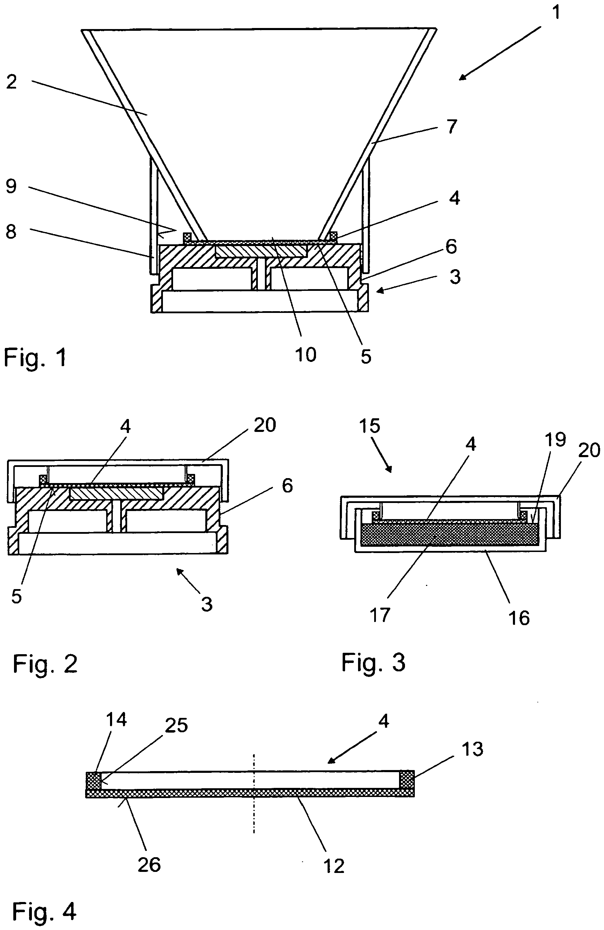

- Figur 1:: eine Seitenansicht einer Filtrationseinheit im Schnitt,
- Figur 2:: eine Seitenansicht des Unterteils von Figur 1 mit Membranfilter und aufgesetztem Deckel einer Nährmedieneinheit im Schnitt,
- Figur 3:: eine Seitenansicht im Schnitt einer Nährmedieneinheit mit aufgesetztem Deckel und eingebrachtem Membranfilter,
- Figur 4:: eine Seitenansicht des Membranfilters von Figur 1 im Schnitt und vergrößerter Darstellung,
- Figur 5:: eine Seitenansicht des Deckels von Figur 3 mit Membranfilter im Schnitt und vergrößerter Darstellung,
- Figur 6:: eine Seitenansicht einer Nährmedieneinheit mit konvex gewölbtem Nährboden und Stützring im Schnitt und vergrößerter Darstellung,
- Figur 7:: eine Untersicht auf den Deckel von Figur 5 in vergrößerter Darstellung und
- Figur 8:: eine Seitenansicht einer weiteren Filtrationsvorrichtung im Schnitt.

### Beschreibung eines Ausführungsbeispiels

Eine Filtrationseinheit 1 besteht im Wesentlichen aus einem Aufsatz 2, einem Unterteil 3 und einem Membranfilter 4.

Das Unterteil 3 weist eine Filterunterstützung 5 auf, auf der der Membranfilter 4 abgelegt ist. Das Unterteil 3 weist weiterhin einen Absatz 6 zur Aufnahme des Aufsatzes 2 auf.

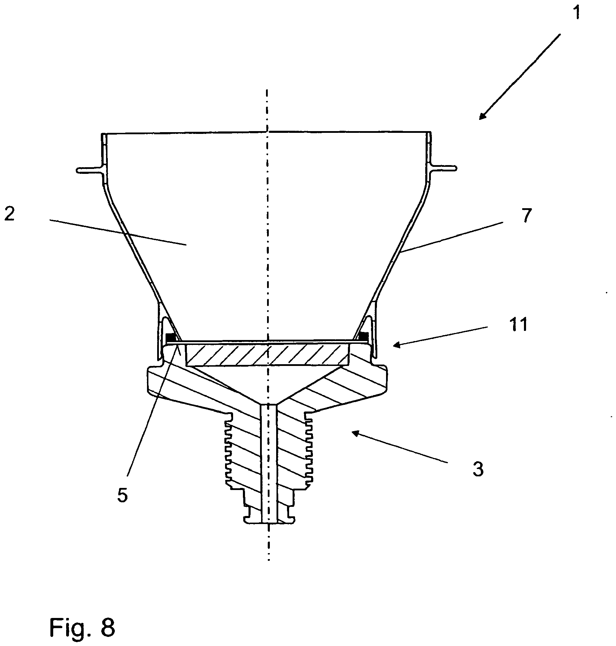

Der Aufsatz 2 ist als ein Trichter 7 ausgebildet, der an seinem in vertikaler Richtung unterem Ende einen zylindrischen Ansatz 8 aufweist, der mit seiner inneren Mantelfläche 9 über den Absatz 6 des Unterteils 3 greift. Im Innenbereich des zylindrischen Ansatzes 8 weist der Trichter 7 eine Auslassöffnung 10, die als Anschlag gegenüber dem Unterteil 3 bzw. gegenüber dem auf dem Unterteil 3 angeordneten Membranfilter 4 dient, auf. Die Fixierung des Trichters 7 auf dem Unterteil 3 erfolgt über eine Klemmung oder entsprechend Figur 8 über eine Rastung 11.

Der Membranfilter 4 besteht aus einer Membran 12 mit einem Verstärkungsrand 13. Zur Bildung des Verstärkungsrandes 13 ist auf die ebene Membran 12 ein Verstärkungsring 14 aufgeklebt oder durch ein Siegelverfahren aufgebracht. Der Membranfilter 4 wird vom Aufsatz 2 so auf das Unterteil 3 geklemmt, dass der Verstärkungsrand 13 frei von Kontakt zum Trichter 7 oder der inneren Mantelfläche 9 zu liegen kommt, sodass nach der Filtration bei der Abnahme des Aufsatzes 2 der Membranfilter 4 auf dem Unterteil 3 liegen bleibt.

Nach dem Filtrieren der Probe wird der Membranfilter 4 in eine Nährmedieneinheit 15 eingebracht. Die Nährmedieneinheit 15 weist ein schalenförmigen Unterteil 16 auf, das als eine Petrischale oder eine Agarschale ausgebildet sein kann. In dem Unterteil 16 ist ein Nährboden 17, beispielsweise aus Agar, angeordnet.

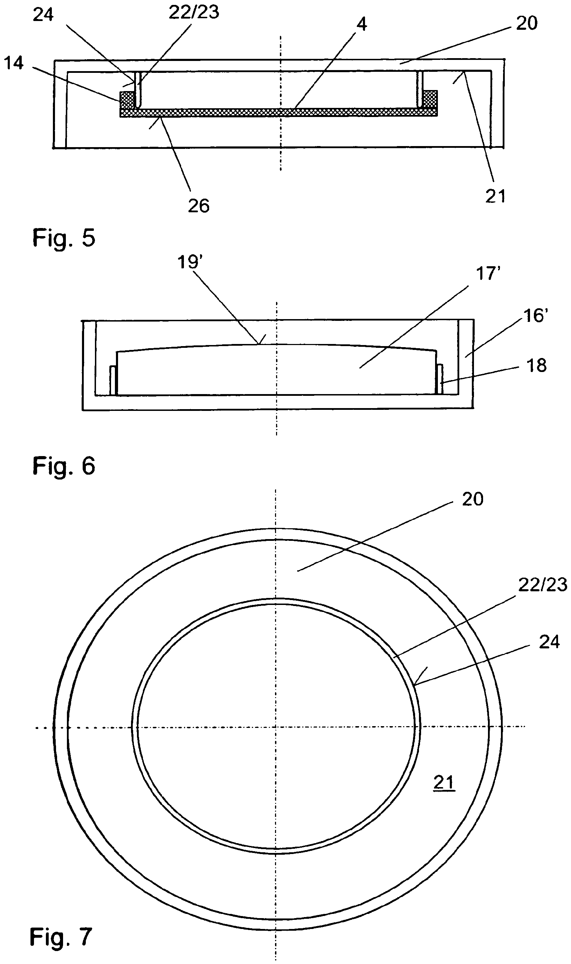

Entsprechend dem Ausführungsbeispiel von Figur 6 weist das Unterteil 16' einen Stützring 18 auf, der den Nährboden 17' in seinem unteren Bereich seitlich abstützt. Im unbelasteten Zustand ist die Oberseite 19, 19' des Nährbodens 17, 17', die in vertikaler Richtung oben liegt, konvex gewölbt, sodass der Nährboden 17, 17' zur Mitte hin etwas dicker ist. Die Nährmedieneinheit 15 weist einen auf das Unterteil 16, 16' aufsetzbaren Deckel 20 auf. Der Deckel 20 weist auf seiner dem Unterteil 16, 16' zugewandten Innenseite 21 ein Klemmteil 22 auf, das als eine ringförmige Innenwandung 23 ausgebildet ist. Das dem Unterteil 16 zugewandte freie Ende der ringformigen Innenwandung 23 ist durch Aufsetzen des Deckels 20 auf dem auf der Filterunterstützung 5 liegenden Membranfilter 4 klemmend innen an den Verstärkungsrand 13 des Membranfilters 4 anlegbar. Bei dem vom Deckel 20 aufgenommenen Membranfilter 4 liegt somit die ringförmige Innenwandung 23 bzw. Außenfläche 24 des Klemmteiles 22 klemmend an der Innenfläche 25 des Verstärkungsrandes 13 an.

Zum Einbringen des Membranfilters 4 in die Nährmedieneinheit 15 wird nach dem Filtrieren der Aufsatz 2 von dem Unterteil 3 abgehoben. Anschließend wird der Deckel 20 von dem Unterteil 16, 16' der Nährmedieneinheit 15 abgenommen und auf den auf der Filterunterstützung 5 liegenden Membranfilter 4 so aufgesetzt, dass das Klemmteil 22 bzw. die ringförmige Innenwandung 23 mit dem Verstärkungsrand 13 bzw. der ringförmigen Innenfläche 25 des Membranfilters 4 sich verklemmt und der Deckel 20 mit dem Membranfilter 4 von der Filterunterstützung 5 abgehoben und auf das Unterteil 16, 16' der Nährmedieneinheit 15 aufgesetzt wird. Dabei wird der Deckel 20 mit der Unterseite 26 des Membranfilters 4 auf die Oberseite 19, 19' des Nährbodens 17, 17' aufgelegt.

## Patentansprüche

1. Filtrationseinheit (1) mit einem auf einer Filterunterstützung (5) eines Unterteiles (3) anordenbaren Membranfilter (4) und einem auf das Unterteil (3) aufsetzbaren und von diesem abnehmbaren Aufsatz (2) mit einem Trichter (7),
**dadurch gekennzeichnet, dass** eine Nähr medieneinheit (15) nach Anspruch 12 vorhanden ist und dass der Membranfilter (4) einen Verstärkungsrand (13) aufweist, dass der Membranfilter (4) durch den Aufsatz (2) ohne Kontakt des Verstärkungsrands (13) zum Trichter (7) auf das Unterteil (3) klemmbar ist, und
dass bei abgenommenem Aufsatz (2) der Membranfilter (4) zur Entnahme mit einem Klemmteil (22) des Deckels (20) der Nährmedieneinheit (15) verklemmbar, von der Filterunterstützung (5) abhebbar und in die Nährmedieneinheit (15) einbringbar ist.

2. Filtrationseinheit nach Anspruch 1,
**dadurch gekennzeichnet,**
**dass** das Klemmteil (22) als eine ringförmige Innenwandung (23) des Deckels (20) ausgebildet ist, deren der Nährmedieneinheit (15) zugewandtes freies Ende klemmend an den Verstärkungsrand (13) anlegbar ist.

3. Filtrationseinheit nach Anspruch 2,
**dadurch gekennzeichnet,**
**dass** das freie Ende der ringförmigen Innenwandung (23) innen an den Verstärkungsrand (13) anlegbar ist.

4. Filtrationseinheit nach einem der Ansprüche 1 bis 3, **dadurch gekennzeichnet,**
**dass** die Nährmedieneinheit (15) ein schalenförmiges Unterteil (16, 16') aufweist,
**dass** der Deckel (20) auf das Unterteil (16, 16') aufsetzbar ist und
**dass** in dem Unterteil (16, 16') ein Nährboden (17, 17') angeordnet ist, auf dessen dem Deckel (20) zugewandter Oberseite (19, 19') der im Deckel (20) verklemmte Membranfilter (4) ablegbar ist.

5. Filtrationsvorrichtung nach Anspruch 4,
**dadurch gekennzeichnet,**
**dass** die Oberseite (19, 19') des Nährbodens (17, 17') konvex gewölbt ist.

6. Filtrationseinheit nach Anspruch 4,
**dadurch gekennzeichnet,**
**dass** der Membranfilter (4) durch einen Überdruck im zwischen ihm und dem Deckel (20) gebildeten Luftraum zu dem Nährboden (17, 17') hin gewölbt ist.

7. Filtrationseinheit nach Anspruch 5 oder 6,
**dadurch gekennzeichnet,**
**dass** nach dem Aufsetzen des Deckels (20) auf das Unterteil (16) der Nährmedieneinheit der Membranfilter (4) und die Oberseite (19, 19') des Nährbodens (17, 17') eben und abstandsfrei aneinanderliegen.

8. Filtrationseinheit nach einem der Ansprüche 4 bis 7,
**dadurch gekennzeichnet,**
**dass** im Unterteil (16') konzentrisch zur Außenwandung ein den Nährboden (17') seitlich in einem unteren Bereich abstützender Stützring (18) angeordnet ist, dessen
Innendurchmesser etwa dem Außendurchmesser des Membranfilters (4) entspricht.

9. Filtrationseinheit nach einem der Ansprüche 1 bis 8,
**dadurch gekennzeichnet,**
**dass** der Nährboden (17, 17') aus Agar ausgebildet ist.

10. Filtrationseinheit nach einem der Ansprüche 1 bis 9,
**dadurch gekennzeichnet,**
**dass** der Membranfilter (4) aus einer ebenen Membran (12) ausgebildet ist, auf der zur Verstärkung ihres Randes auf der der Filterunterstützung (5) abgewandten ersten Seite ein den Verstärkungsrand (13) bildender Verstärkungsring (14) angeordnet ist.

11. Filtrationseinheit nach Anspruch 10,
**dadurch gekennzeichnet,**
**dass** der Verstärkungsring (14) über ein Klebe- oder Siegelverfahren mit der Membran (12) fest verbunden ist.

12. Nährmedieneinheit (15) zur Aufnahme eines Membranfilters (4) einer Filtrationseinheit (1) mit einem mit einem Nährboden (17, 17') gefüllten Unterteil (16, 16') und einem Deckel (20),
wobei der Deckel (20) ein in das Unterteil (16, 16') hineinragendes Klemmteil (22) aufweist, das zur Entnahme des Membranfilters (4) aus der Filtrationseinheit (1) mit einem Verstärkungsrand (13) des Membranfilters (4) verklemmbar ist,
**dadurch gekennzeichnet,**
**dass** das Unterteil (16, 16') einen Stützring (18) aufweist, der den Nährboden (17, 17') in seinem unteren Bereich seitlich abstützt, und dass im unbelasteten Zustand die Oberseite (19, 19') des Nährbodens ((17, 17'), die in vertikaler Richtung oben liegt, konvex gewölbt ist.

13. Verfahren zur mikrobiologischen Untersuchung flüssiger Proben nach vorangegangener Filtration unter Verwendung einer Filtrationseinheit nach einem der vorangegangenen Ansprüche 1 bis 11, bei dem ein Membranfilter (4) durch einen Aufsatz (2) ohne Kontakt eines Verstärkungsrands (13) zu einem Trichter (7) auf ein Unterteil (3) geklemmt wird und nach Entfernen des Aufsatzes (2) zum Aufgießen der flüssigen Probe von einer Filterunterstützung (5) abgehoben und auf einer Oberfläche eines in einem Unterteil (16, 16') einer Nährmedieneinheit (15) angeordneten Nährbodens (17, 17') abgelegt und das Unterteil (16, 16') von einem Deckel (20) abgedeckt wird,
**dadurch gekennzeichnet,**
**dass** der Deckel (20) auf den auf der Filterunterstützung (5) liegenden Membranfilter (4) so aufgesetzt wird, dass ein in dem Deckel (20) angeordnetes Klemmteil (22) mit einem Verstärkungsrand (13) des Membranfilters (4) sich verklemmt, und
**dass** der Deckel (20) mit dem Membranfilter (4) von der Filterunterstützung (5) abgehoben und auf das schalenförmige Unterteil (16, 16') so aufgesetzt wird, dass die dem Deckel (20) abgewandte Unterseite (26) des Membranfilters (4) auf der dem Deckel (20) zugewandten Oberseite (19, 19') des Nährbodens (17, 17') aufliegt.

14. Verfahren nach Anspruch 13,
**dadurch gekennzeichnet,**
**dass** der Deckel (20) mit dem geklemmten Membranfilter (4) auf eine Petrischale mit einer Agarplatte als Nährboden (17, 17') aufgesetzt wird.

## Claims

1. Filtration unit (1) with a membrane filter (4), which can be arranged on a filter support (5) of a bottom part (3), and a top part (2), which can be placed on the bottom part (3) and removed therefrom, with a funnel (7), **characterised in that** a culture medium unit (15) according to claim 12 is present and that the membrane filter (4) has a reinforcing edge (13), that the membrane filter (4) can be clamped on the bottom part (3) by the top part (2) without contact of the reinforcing edge (13) with the funnel (7) and that when the top part (2) is removed the membrane filter (4) can for removal be clamped by a clamping part (22) of the cover (20) to the culture medium unit (15) and can be lifted off the filter support (5) and introduced into the culture medium unit (15).

2. Filtration unit according to claim 1, **characterised in that** the clamping part (22) is constructed as an annular inner wall (23) of the cover (20), the free end - which faces the culture medium unit (15) - of which can be placed with clamping action at the reinforcing edge (13).

3. Filtration unit according to claim 2, **characterised in that** the free end of the annular inner wall (23) can be placed at the inside at the reinforcing edge (13).

4. Filtration unit according to any one of claims 1 to 3, **characterised in that** the culture medium unit (15) has a bowl-shaped lower part (16, 16'), that the cover (20) can be placed on the lower part (16, 16') and that arranged in the lower part (16, 16') is a culture medium (17, 17') on which the upper side (19, 19') - which faces the cover (20) - of which the membrane filter (4) clamped in the cover (20) can be placed.

5. Filtration device according to claim 4, **characterised in that** the upper side (19, 19') of the culture medium (17, 17') is convexly curved.

6. Filtration unit according to claim 4, **characterised in that** the membrane filter (4) is curved towards the culture medium (17, 17') by an excess pressure in the air space formed between it and the cover (20).

7. Filtration unit according to claim 5 or 6, **characterised in that** after the placing the cover (20) on the lower part (16) of the culture medium unit the membrane filter (4) and the upper side (19, 19') of the culture medium (17, 17') lie flatly one on the other without spacing.

8. Filtration unit according to any one of claims 4 to 7, **characterised in that** arranged in the lower part (16') concentrically with respect to the outer wall is a support ring (18) which supports the culture medium (17') laterally in a lower region and the inner diameter of which approximately corresponds with the outer diameter of the membrane filter (4).

9. Filtration unit according to any one of claims 1 to 8, **characterised in that** the culture medium (17, 17') is formed from agar.

10. Filtration unit according to any one of claims 1 to 9, **characterised in that** the membrane filter (4) is formed from a planar membrane (12) of which for reinforcement of the edge thereof a reinforcing ring (14) forming the reinforcing edge (13) is arranged on the first side remote from the filter support (5).

11. Filtration unit according to claim 10, **characterised in that** the reinforcing ring (14) is fixedly connected with the membrane (12) by way of a glueing of sealing process.

12. Culture medium unit (15) for receiving a membrane filter (4) of a filtration unit (1), with a lower part (16, 16') filled with a culture medium (17, 17') and with a cover (20), wherein the cover (20) comprises a clamping part (22) which protrudes into the lower part (16, 16') and which for removal of the membrane filter (4) from the filtration unit (1) can be clamped to a reinforcing edge (13) of the membrane filter (4), **characterised in that** the lower part (16, 16') has a support ring (18) which laterally supports the culture medium (17, 17') in its lower region and that in the unloaded state the upper side (19, 19') of the culture medium (17, 17'), which lies at the top in vertical direction, is convexly curved.

13. Method for microbiological examination of liquid samples after preceding filtration with use of a filtration unit according to any one of the preceding claims 1 to 11, in which a membrane filter (4) is clamped on a bottom part (3) by a top part (2) without contact of a reinforcing edge (13) with a funnel (7) and after removal of the top part (2) is lifted up for pouring on the liquid sample from a filter support (5) and is placed on a surface of a culture medium (17, 17') arranged in a lower part (16, 16') of a culture medium unit (15) and the lower part (16, 16') is covered by a cover (20), **characterised in that** the cover (20) is so placed on the membrane filter (4) lying on the filter support (5) that a clamping part (22) arranged in the cover (20) clamps with a reinforcing edge (13) of the membrane filter (4) and that the cover (20) together with the membrane filter (4) is lifted off the filter support (5) and so placed on the bowl-shaped lower part (16, 16') that the underside (26), which is remote from the cover (20), of the membrane filter (4) rests on the upper side (19, 19'), which faces the cover (20), of the culture medium (17, 17').

14. Method according to claim 13, **characterised in that** the cover (20) together with the clamped membrane filter (4) is placed on a Petri dish with an agar plate as culture medium (17, 17').

## Revendications

1. Unité de filtration (1) comprenant une membrane filtrante (4) disposée sur la partie inférieure (3) d'un support de filtre (5) et comprenant une pièce supérieure (2) avec un entonnoir (7) pouvant être posée sur la partie inférieure (3) et pouvant en être enlevée,
**caractérisée en ce qu'**une unité de milieu de culture (15) selon la revendication 12 est présente et que la membrane filtrante (4) présente un bord de renfort (13), que la membrane filtrante (4) peut être serrée contre le partie inférieure (3) par l'intermédiaire de la pièce supérieure (2), sans contact du bord de renfort (13) avec l'entonnoir (7), et
que lorsque la pièce supérieure (2) est enlevée pour le prélèvement, la membrane filtrante (4) peut être serrée à l'aide d'une pièce de serrage (22) du couvercle (20) de l'unité de milieu de culture (15), peut être soulevée du support de filtre (5) et peut être introduite dans l'unité de milieu de culture (15).

2. Unité de filtration selon la revendication 1 **caractérisée en ce**
**que** la pièce de serrage (22) est conçue dans la paroi interne du couvercle (20) sous la forme d'un anneau (23) dont l'extrémité libre orientée vers l'unité du milieu de culture (15) peut se plaquer en se serrant contre le bord de renfort (13).

3. Unité de filtration selon la revendication 2
**caractérisée en ce**
**que** l'extrémité libre de la paroi interne en forme d'anneau (23) peut se plaquer à l'intérieur contre le bord de renfort (13).

4. Unité de filtration selon l'une des revendications de 1 à 3
**caractérisée en ce**
**que** l'unité de milieu de culture (15) présente une partie inférieure (16, 16') en forme de coupe,
**que** le couvercle (20) peut être posé sur la partie inférieure (16, 16') et
**qu'**un milieu de culture (17, 17') est disposé dans la partie inférieure (16, 16'), milieu sur lequel la face supérieure (19, 19'), orientée vers le couvercle (20), de la membrane filtrante (4) serrée dans le dans couvercle (20) peut être appliquée.

5. Unité de filtration selon la revendication 4
**caractérisée en ce**
**que** la face supérieure (19, 19') du milieu de culture (17, 17') est bombée de manière convexe.

6. Unité de filtration selon la revendication 4
**caractérisée en ce**
**que** la membrane filtrante (4) est bombée par rapport au milieu de culture (17, 17') du fait d'une surpression dans l'espace d'air formé entre elle et le couvercle (20).

7. Unité de filtration selon les revendications 5 ou 6
**caractérisée en ce**
**qu'**après avoir posé le couvercle (20) sur la partie inférieure (16) de l'unité du milieu de culture, la membrane filtrante (4) et la face supérieure (19, 19') du milieu de culture (17, 17') sont disposées l'une sur l'autre à plat et sans espace entre elles.

8. Unité de filtration selon l'une des revendications de 4 à 7
**caractérisée en ce**
**qu'**un milieu de culture (17') est disposé sur le pourtour d'un anneau support (18) s'appuyant sur la zone inférieure dans la partie inférieure (16') de manière concentrique par rapport à la paroi externe, anneau support dont le diamètre intérieur correspond à peu près au diamètre extérieur de la membrane filtrante (4).

9. Unité de filtration selon l'une des revendications de 1 à 8
**caractérisé en ce**
**que** le milieu de culture (17, 17') est constitué d'agar.

10. Unité de filtration selon l'une des revendications de 1 à 9
**caractérisée en ce**
**que** la membrane filtrante (4) est constituée d'une membrane plane (12) sur laquelle un anneau formant un renfort (14), façonné sur la première face du support de filtre (5) opposée au bord de renfort (13), est disposé pour le renforcement de son bord.

11. Unité de filtration selon la revendication 10
**caractérisée en ce**
**que** l'anneau de renfort (14) est relié solidement avec la membrane (12) par un procédé de collage ou de scellement.

12. Unité de milieu de culture (15) pour l'admission d'une membrane filtrante (4) d'une unité de filtration (1) comprenant une partie inférieure (16, 16') remplie avec un milieu de culture (17, 17') et un couvercle (20),
le couvercle (20) présentant une pièce de serrage (22) rentrant dans la partie inférieure (16, 16'), qui peut être serrée avec un anneau de renfort (13) de la membrane filtrante (4) pour le prélèvement de la membrane filtrante (4) de l'unité de filtration (1),
**caractérisée en ce**
**que** la partie inférieure (16, 16') présente un anneau support (18) qui soutient latéralement dans sa zone inférieure le milieu de culture (17, 17'), et que dans la position non sollicitée, la face supérieure (19, 19') du milieu de culture (17, 17'), qui est située en haut dans la direction verticale, est bombée de manière convexe.

13. Procédé pour l'examen microbiologique d'échantillons liquides selon la filtration précédente moyennant l'emploi d'une unité de filtration selon l'une des revendications précédentes de 1 à 11, où une membrane filtrante (4) est serrée contre une partie inférieure (3) par l'intermédiaire d'une pièce supérieure (2), sans contact d'un bord de renfort (13) avec un entonnoir (7), et où, après l'enlèvement de la pièce supérieure (2) pour le versement de l'échantillon liquide, est soulevée d'un support de filtre (5) et est posée sur la surface d'un milieu de culture (17, 17') disposé dans la partie inférieure (16, 16') d'une unité de milieu de culture (15) et où la partie inférieure (16, 16') est recouverte d'un couvercle (20),
**caractérisé en ce**
**que** le couvercle (20) est disposé sur la membrane filtrante (4) posée sur le support de filtre (5) de telle manière qu'une pièce de serrage (22) agencée dans le couvercle (20) est serrée avec un bord de renfort (13) de la membrane filtrante (4), et
**que** le couvercle (20) peut être soulevé avec la membrane filtrante (4) du support de filtre (5) et peut être posé sur la partie inférieure (16, 16') en forme de coupe de telle manière que la face inférieure (26) de la membrane filtrante (4), opposée au couvercle (20), repose sur la face supérieure (19, 19') du milieu de culture (17, 17'), orientée vers le couvercle (20).

14. Procédé selon la revendication 13
**caractérisé en ce**
**que** le couvercle (20) est posé avec la membrane filtrante (4) serrée sur une boîte de Pétri comprenant une plaque d'agar comme milieu de culture (17, 17').
